(19) Europäisches Patentamt / European Patent Office / Office européen des brevets

(11) **EP 2 337 547 B1**

(12) **EUROPEAN PATENT SPECIFICATION**

(45) Date of publication and mention
of the grant of the patent:
**09.10.2013 Bulletin 2013/41**

(51) Int Cl.:
*A61K 8/27* (2006.01)   *A61K 8/29* (2006.01)
*A61K 8/73* (2006.01)   *A61Q 17/04* (2006.01)

(21) Application number: **09748885.2**

(22) Date of filing: **20.10.2009**

(86) International application number:
**PCT/US2009/061250**

(87) International publication number:
**WO 2010/048128 (29.04.2010 Gazette 2010/17)**

(54) **HYDROXYPROPYL METHYLCELLULOSE-CONTAINING SUNSCREEN COMPOSITIONS AND METHODS OF USE**

HYDROXYPROPYLMETHYLCELLULOSE ENTHALTENDE
SONNENSCHUTZZUSAMMENSETZUNGEN UND ANWENDUNGSVERFAHREN

COMPOSITIONS D'ÉCRAN SOLAIRE CONTENANT DE
L'HYDROXYPROPYLMÉTHYLCELLULOSE ET PROCÉDÉS D'UTILISATION

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **22.10.2008 US 107507 P**

(43) Date of publication of application:
**29.06.2011 Bulletin 2011/26**

(73) Proprietor: **Dow Global Technologies LLC
Midland, MI 48674 (US)**

(72) Inventor: **FLETCHER, Robert, B.
Midland
MI 48642 (US)**

(74) Representative: **Raynor, John
Beck Greener
Fulwood House
12 Fulwood Place
London WC1V 6HR (GB)**

(56) References cited:
**US-B1- 6 261 713**

• **ANONYMOUS/ THE DOW CHEMICAL COMPANY:
"Chemistry of Methocel Cellulose Ethers",
INTERNET CITATION, 13 March 2008
(2008-03-13), pages 1-2, XP002550463, Retrieved
from the Internet: URL:/http://www.dow.com/
dowexcip ients/products/chemistry/
methocel.htm [retrieved on 2009-10-14]**

**Description**

**Cross Reference to Related Applications**

This application claims the benefit of U.S. Provisional Application No. 61/107,507, filed October 22, 2008.

**Field**

The present invention relates to sunscreen compositions.

**Background**

The balance between organic and inorganic sunscreens in the sun care market is tilting toward inorganic sunscreens in response to regulatory changes and consumer demands. Many countries now require quantifiable UVA protection on sunscreen labels, and zinc oxide and titanium dioxide are two of the few filters that offer UVA protection. However, achieving today's UVA and UVB label claims with inorganic sunscreens alone can be challenging because high concentrations of inorganic sunscreens can be expensive and/or can cause an undesirable white appearance when applied to the skin.

Accordingly, there is a need for sunscreen boosters which will help achieve high SPF (sun protection factor) and high UVA protection with lower inorganic concentrations and without the need for additional organic sunscreens.

**Summary**

In one embodiment, the present invention provides sunscreen compositions, comprising inorganic metal oxide sunscreen particles and hydroxypropyl methylcellulose, provided that the hydroxypropyl methylcellulose has a $DS_{methoxyl}$ of less than 1.7.

In another embodiment, the present invention provides methods of boosting the SPF of a sunscreen composition comprising inorganic metal oxide sunscreen particles, comprising including hydroxypropyl methylcellulose in the sunscreen composition, provided that the hydroxypropyl methylcellulose has a $DS_{methoxyl}$ of less than 1.7.

In another embodiment, the present invention provides methods of boosting the SPF of a sunscreen composition comprising inorganic metal oxide sunscreen particles, comprising selecting a hydroxypropyl methylcellulose with a $DS_{methoxyl}$ of less than 1.7, and incorporating the hydroxypropyl methylcellulose in the sunscreen composition.

**Detailed Description**

In one embodiment, the present invention provides sunscreen compositions, comprising inorganic metal oxide sunscreen particles and hydroxypropyl methylcellulose, provided that the hydroxypropyl methylcellulose has a $DS_{methoxyl}$ of less than 1.7.

Preferably, the inorganic metal oxide sunscreen particles are selected from zinc oxide (ZnO), titanium dioxide ($TiO_2$), or mixtures thereof. In one embodiment, the inorganic metal oxide sunscreen particles are pigment grade zinc oxide or pigment grade titanium dioxide.

In one embodiment, the inorganic metal oxide sunscreen particles are transparent zinc oxide or transparent titanium dioxide. Most inorganic metal oxide sunscreen particles used in a sunscreen produce a cosmetically undesirable white appearance caused by light scattering. Thus, the term "transparent inorganic metal oxide sunscreen particles," as used  herein has a special meaning, referring to inorganic metal oxide sunscreen particles produced by a variety of processing conditions which render the inorganic metal oxide compositions as clear, or transparent, upon application. In other words, these specially processed inorganic metal oxide compositions do not appear white once applied, hence the moniker of transparent.

Examples of transparent zinc oxide are disclosed in, for example, US Patent Nos. 5,223,250, 5,372,805, 5,573,753, 5,587,148, and 5,876,688. One example of a transparent zinc oxide is commercially available under the tradename Z-COTE from BASF Corporation (Germany). Another example of transparent zinc oxide is commercially available under the tradename ZINCLEAR IM from Antaria Limited (Australia). Another example of transparent zinc oxide is commercially available under the tradename Z-CLEAR from Actifirm (USA).

Examples of transparent titanium dioxide are disclosed in, for example, US Patent Nos. 5,573,753, 5,733,895, and 7,390,355. Examples of transparent titanium dioxide are commercially available under the tradenames TIPAQUE and TTO-51(A) from Ishihara Sangyo Kaisha, Ltd. (Japan). Another example of a transparent titanium dioxide is commercially available under the tradename T-COTE from BASF Corporation (Germany). Another example of transparent titanium dioxide is commercially available under the tradename UFTR from Miyoshi Kasei (Japan). Another example of transparent titanium dioxide is commercially available under the tradename SOLAVEIL CLARUS from Uniqema (Great Britain).

The transparent inorganic metal oxide sunscreen particles are selected from transparent zinc oxide, titanium dioxide, or mixtures thereof. In one embodiment, the transparent zinc oxide sunscreen particles are present in an amount from 0.1% to 50%, and preferably 1% to 25%. The transparent titanium dioxide sunscreen particles are present in an amount 0.1% to 50%, and preferably 1% to 25%.

Hydroxylpropyl methylcellulose is generally available under the tradename METHOCEL E, F, J, and K (The Dow Chemical Company). The polymeric backbone of cellulose is a repeating structure of anhydroglucose units. Treatment of cellulosic fibers with caustic solution, followed by methyl chloride and propylene oxide, yields cellulose ethers substituted with methoxy groups and hydroxypropyl groups. The term "DS" refers to the degree of methoxyl substitution per anhydroglucose unit. The term "MS" refers to the degree of hydroxypropoxyl substitution per anhydroglucose unit. Each grade is differentiated by the methoxy and hydroxypropyl substitution on the polymeric backbone.

Hydroxylpropyl methylcellulose useful in the present invention has a $DS_{methoxyl}$ of less than 1.7. Preferably, the DS is less than about 1.6, preferably less than about 1.5, preferably less than about 1.4. Hydroxylpropyl methylcelluloses with a $DS_{methoxyl}$ of less than 1.7 are generally available under the tradename METHOCEL J and K from The Dow Chemical Company.

In one embodiment, the hydroxypropyl methylcellulose has a viscosity at 2% concentration in water at 20°C, of about 1 cps to about 100,000 cps, preferably about 5 cps to about 30cps, most preferably about 15 cps.

The hydroxypropyl methylcellulose is present in an amount from 0.05% to 15%, and preferably 0.1% to 5%.

Compositions of the present invention can further incorporate other ingredients known in the art of sunscreen formulations including at least one of organic suncare actives, cosmetically acceptable emollients, moisturizers, conditioners, oils, suspending agents, opacifiers/pearlizers, surfactants, emulsifiers, preservatives, rheology modifiers, colorants, pH adjustors, propellants, reducing agents, anti-oxidants, fragrances, foaming or de-foaming agents, tanning agents, insect repellants, or biocides. Preferably, sunscreen compositions of the present invention include at least one of a humectant, a surfactant, an emollient, and a preservative.

In another embodiment, the present invention provides methods of boosting the SPF of a sunscreen composition comprising inorganic metal oxide sunscreen particles, comprising including hydroxypropyl methylcellulose in the sunscreen composition, provided that the hydroxypropyl methylcellulose has a $DS_{methoxyl}$ of less than 1.7.

In another embodiment, the present invention provides methods of boosting the SPF of a sunscreen composition comprising inorganic metal oxide sunscreen particles, comprising selecting a hydroxypropyl methylcellulose with a $DS_{methoxyl}$ of less than 1.7, and incorporating the hydroxypropyl methylcellulose in the sunscreen composition.

## Examples

The following examples are for illustrative purposes only and are not intended to limit the scope of the present invention. All percentages are by weight unless otherwise specified.

### Example 1

Exemplary sunscreen compositions contain the components recited in TABLE 1.

**TABLE** 1

| Component | Batch 1 | Batch 2 |
|---|---|---|
| METHOCEL K4M hydroxypropyl methylcellulose | 1 | 0 |
| METHOCEL J5MS hydroxypropyl methylcellulose | 0 | 1 |
| Deionized Water | 59.1 | 59.1 |
| Propylene Glycol | 1 | 1 |
| GLUCAM E-10 methyl gluceth-10 | 1 | 1 |
| LIQUAPAR OPTIMA phenoxyethanol, methylparaben, isopropylparaben, isobutylparaben, and butylparaben | 0.9 | 0.9 |
| PEG 40 stearate | 1 | 1 |
| gylceryl stearate | 1 | 1 |
| PROMULGEN D cetearyl alcohol and ceteareth-20 | 3 | 3 |

(continued)

| Component | Batch 1 | Batch 2 |
|---|---|---|
| DC 200 Dimethicone | 2 | 2 |
| ZINCLEAR IM_50 AB (50% active) transparent zinc oxide | 30 | 30 |
| Citric Acid | q.s | q.s |

In Batch 1, a 4% stock solution is prepared by slowly adding METHOCEL K4M to about 75°C water. For Batch 2, a 4% stock solution is prepared by mixing METHOCEL J5MS with cold water and increasing the pH by adding NaOH, under agitation. Beyond the preparation of the stock solutions the METHOCEL stock solution is prepared the preparation of Batch 1 and 2 is the same. Once the METHOCEL is homogeneously mixed, the dispersion is allowed to cool to room temperature. Additional water, propylene glycol, methyl gluceth, and OPTIMA are combined at room temperature, and then the 4% METHOCEL stock is added to form a first mixture. This mixture is then heated to about 75°C to dissolve all the solid ingredients.

PEG 40 stearate, gylceryl stearate, cetearyl alcohol and ceteareth-20, dimethicone, and transparent zinc oxide are combined to form a second mixture, and this mixture is then heated to about 75°C to dissolve all the solid ingredients.

The first mixture and second mixture are combined while both are still relatively hot, i.e., about 75°C, and mixed using a Silverson IKA Homogenizer. Citric acid is added drop-wise until a pH of about 7.5 to about 8 is attained.

### Example 2 (Comparative)

Comparative sunscreen compositions contain the components recited in TABLE 2.

**TABLE 2**

| Component | Comparative Batch A | Comparative Batch B |
|---|---|---|
| METHOCEL hydroxypropyl cellulose F4M | 1 | 0 |
| METHOCEL hydroxypropyl cellulose E4M | 0 | 1 |
| Deionized Water | 59.1 | 59.1 |
| Propylene Glycol | 1 | 1 |
| GLUCAM E-10 methyl gluceth-10 | 1 | 1 |
| LIQUAPAR OPTIMA phenoxyethanol, methylparaben, isopropylparaben, isobutylparaben, and butylparaben | 0.9 | 0.9 |
| PEG 40 stearate | 1 | 1 |
| gylceryl stearate | 1 | 1 |
| PROMULGEN D cetearyl alcohol and ceteareth-20 | 3 | 3 |
| DC 200 Dimethicone | 2 | 2 |
| ZINCLEAR IM_50 AB (50% active) transparent zinc oxide | 30 | 30 |
| Citric Acid | q.s | q.s |

The composition is prepared substantially as described above with respect to Example 1. Both the METHOCEL E4M and METHOCEL F4M are prepared using the hot water method described in Example 1, Batch 1.

### Example 3

Sunscreen compositions prepared substantially according to the protocols of Examples 1 and 2 were prepared and their SPF values determined and recited in TABLE 3.

TABLE 3

|  | Batch 1 | Batch 2 | Comparative Batch A | Comparative Batch B |
|---|---|---|---|---|
| SPF | 36.7 ± 7 | 38.2 ± 2 | 27.6 ± 3.4 | 32.1 ± 4.4 |

The SPF was determined using an *in vitro* technique substantially according to the following protocol:

Initially, the weight of a roughened PMMA substrate (purchased from SCHÖNBERG GmbH & Co. KG, Hamburg / Germany,) is measured. The batch to be tested is then deposited on the substrate and then quickly leveled with a 7 micron draw down bar to achieve a thin, uniform layer. The layer is allowed to dry for about 20 minutes, and the weight of the substrate plus dry uniform layer is determined.

The UV absorption of dry uniform layer is measured using a LABSPHERE 1000S spectrometer at multiple points on the layer.

The percent solids of the layer is measured using a METTLER LP 16 solids analyzer. Using the weight of the dry film, and the solids content of the layer, the weight, and consequently the density of the original wet layer immediately after deposition can be calculated. Using this information, the SPF can be calculated by the following equation:

$$SPF = \frac{\int_{290nm}^{400nm} E(\lambda)S(\lambda)\partial\lambda}{\int_{290nm}^{400nm} E(\lambda)S(\lambda)10^{(-A(\lambda))}\partial\lambda}$$

Where $E(\lambda)$ = spectral irradiance of the Standard Sun Spectrum; $S(\lambda)$ = erythemal action spectrum at wavelength $\lambda$; and $A(\lambda)$ = corrected spectral absorbance at wavelength $\lambda$ (a correction factor is calculated to extrapolate the data to establish what the absorbance would be at a wet layer density of 2.0 mg/cm$^2$ (using the original wet layer immediately after deposition).

The results in TABLE 3 are averages of at least 27 measurements for each batch. The present invention results in SPF boosts of 14%, and 19% as compared to a composition with METHOCEL E4M - Comparative Batch B.

**Example 4**

Exemplary sunscreen compositions contain the components recited in TABLE 4.

TABLE 4

| Component | Batch 4 | Batch 5 | Batch 6 |
|---|---|---|---|
| METHOCEL J75MS hydroxypropyl methylcellulose | 1 | 2 | 1 |
| Deionized Water | 59.1 | 58.1 | 59.1 |
| Propylene Glycol | 1 | 1 | 1 |
| GLUCAM E-10 methyl gluceth-10 | 1 | 1 | 1 |
| LIQUAPAR OPTIMA phenoxyethanol, methylparaben, isopropylparaben, isobutylparaben, and butylparaben | 0.9 | 0.9 | 0.9 |
| PEG 40 stearate | 1 | 1 | 1 |
| gylceryl stearate | 1 | 1 | 1 |
| PROMULGEN D cetearyl alcohol and ceteareth-20 | 3 | 3 | 3 |
| DC 200 Dimethicone | 2 | 2 | 2 |
| T-Oleo (33% Active) transparent titanium dioxide | 0 | 0 | 30 |
| ZINCLEAR IM_50 AB (50% active) transparent zinc oxide | 30 | 30 | 0 |
| Citric Acid | q.s | q.s | q.s |

The compositions are prepared substantially as described above with respect to Example 1.

**Example** 5

Sunscreen compositions prepared substantially according to the protocols of Example 4 were prepared and their SPF values determined and recited in TABLE 5 as percent SPF improvement from a composition with no hydroxypropyl methylcellulose.

**TABLE 5**

|  | Batch 4 | Batch 5 | Batch 6 |
|---|---|---|---|
| SPF Boost | 24% | 65% | 43% |

The SPF was determined using an *in vivo* technique substantially according to the following protocol:

A portion of subject's back (without sunscreen) is exposed for incremental time intervals to a solar simulator, usually a 150-watt Xenon Arc Berger Solar Ultraviolet Simulator (Solar Light Co., Philadelphia, PA) with filters which mimic the solar-like spectrum (UVB: 290 to 320 nanometers and UVA: 320 to 400 nanometers). The subject is exposed until the skin is barely pink to the eye of a trained observer.

Then, on an adjacent area of the subject's back that was not exposed, 2mg/cm$^2$ of the test sunscreen is applied evenly and left to dry for ~15 minutes. This area is then exposed to the solar simulator for incremental time intervals until the skin is barely pink to the eye of the aforesaid observer. The exposure duration to obtain the pink color (Minimal Erythemal Dose (MED)) for the protected and unprotected skin are then ratioed to obtain the in vivo SPF using the equation, below:

$$SPF = \frac{(MED(Protected))}{(MED(Unprotected))}$$

It is understood that the present invention is not limited to the embodiments specifically disclosed and exemplified herein. Various modifications of the invention will be apparent to those skilled in the art. Such changes and modifications may be made without departing from the scope of the appended claims. Moreover, each recited range includes all combinations and subcombinations of ranges, as well as specific numerals contained therein. Additionally, the disclosures of each patent, patent application, and publication cited or described in this document are hereby incorporated herein by reference, in their entireties.

**Claims**

1.  A sunscreen composition, comprising:

    inorganic metal oxide sunscreen particles selected from transparent zinc oxide in an amount of from 0.1% to 50% by weight of the composition, transparent titanium dioxide in an amount of from 0.1% to 50% by weight of the composition, or mixtures thereof; and
    hydroxypropyl methylcellulose, provided that the hydroxypropyl methylcellulose has a DS$_{methoxyl}$ of less than 1.7, wherein, the hydroxypropyl methylcellulose is present in an amount of from 0.05% to 15% by weight of the composition;
    wherein the term "transparent" is used herein to mean that the particles are transparent upon application.

2.  The sunscreen composition of Claim 1, wherein the transparent zinc oxide sunscreen particles are present in an amount of from 1% to 25%.

3.  The sunscreen composition of Claim 1, wherein the transparent titanium dioxide sunscreen particles are present in an amount of from 1% to 25%.

4.  The sunscreen composition of Claim 1, wherein the hydroxypropyl methylcellulose is present in an amount of from 0.1% to 5%.

5. The sunscreen composition of Claim 1, wherein the hydroxypropyl methylcellulose has a $DS_{methoxyl}$ of less than 1.6, preferably less than 1.5, preferably less than 1.4.

6. The sunscreen composition of Claim 1, wherein the hydroxypropyl methylcellulose has a viscosity at 2% in water of 1 cps to 100,000 cps, preferably 15 cps.

7. The sunscreen composition of Claim 1, further comprising a humectant.

8. The sunscreen composition of Claim 1, further comprising a surfactant.

9. The sunscreen composition of Claim 1, further comprising an emollient.

10. The sunscreen composition of Claim 1, further comprising a preservative.

11. The sunscreen composition of Claim 1, further comprising at least one organic suncare active.

12. A method of boosting the SPF of a sunscreen composition comprising inorganic metal oxide sunscreen particles selected from transparent zinc oxide, transparent titanium dioxide, or mixtures thereof, comprising:

   a) including hydroxypropyl methylcellulose in the sunscreen composition, provided that the hydroxypropyl methylcellulose has a $DS_{methoxyl}$ of less than 1.7; or
   b) selecting a hydroxypropyl methylcellulose with a $DS_{methoxyl}$ of less than 1.7, and incorporating the hydroxypropyl methylcellulose in the sunscreen composition.

## Patentansprüche

1. Eine Sonnenschutzzusammensetzung, die Folgendes beinhaltet:

   anorganische Metalloxid-Sonnenschutzpartikel, ausgewählt aus transparentem Zinkoxid in einer Menge von 0,1 Gew.-% bis 50 Gew.-% der Zusammensetzung, transparentem Titandioxid in einer Menge von 0,1 Gew.-% bis 50 Gew.-% der Zusammensetzung oder Mischungen davon; und
   Hydroxypropylmethylcellulose, vorausgesetzt, dass die Hydroxypropylmethylcellulose einen $DS_{Methoxyl}$ von weniger als 1,7 aufweist,
   wobei die Hydroxypropylmethylcellulose in einer Menge von 0,05 Gew.-% bis 15 Gew.-% der Zusammensetzung vorliegt;
   wobei der Begriff "transparent" hier so verwendet wird, dass er bedeutet, dass die Partikel bei Auftragung transparent sind.

2. Sonnenschutzzusammensetzung gemäß Anspruch 1, wobei die transparenten Zinkoxid-Sonnenschutzpartikel in einer Menge von 1 % bis 25 % vorliegen.

3. Sonnenschutzzusammensetzung gemäß Anspruch 1, wobei die transparenten Titandioxid-Sonnenschutzpartikel in einer Menge von 1 % bis 25 % vorliegen.

4. Sonnenschutzzusammensetzung gemäß Anspruch 1, wobei die Hydroxypropylmethylcellulose in einer Menge von 0,1 % bis 5 % vorliegt.

5. Sonnenschutzzusammensetzung gemäß Anspruch 1, wobei die Hydroxypropylmethylcellulose einen $DS_{Methoxyl}$ von weniger als 1,6, vorzugsweise weniger als 1,5, vorzugsweise weniger als 1,4 aufweist.

6. Sonnenschutzzusammensetzung gemäß Anspruch 1, wobei die Hydroxypropylmethylcellulose bei 2 % in Wasser eine Viskosität von 1 cP bis 100 000 cP, vorzugsweise 15 cP aufweist.

7. Sonnenschutzzusammensetzung gemäß Anspruch 1, die ferner ein Feuchthaltemittel beinhaltet.

8. Sonnenschutzzusammensetzung gemäß Anspruch 1, die ferner einen grenzflächenaktiven Stoff beinhaltet.

**9.** Sonnenschutzzusammensetzung gemäß Anspruch 1, die ferner ein Emolliens beinhaltet.

**10.** Sonnenschutzzusammensetzung gemäß Anspruch 1, die ferner ein Konservierungsmittel beinhaltet.

**11.** Sonnenschutzzusammensetzung gemäß Anspruch 1, die ferner mindestens einen organischen Sonnenpflegeaktivstoff beinhaltet.

**12.** Ein Verfahren zum Erhöhen des LSF einer Sonnenschutzzusammensetzung, die anorganische Metalloxid-Sonnenschutzpartikel, ausgewählt aus transparentem Zinkoxid, transparentem Titandioxid oder Mischungen davon, beinhaltet, beinhaltend:

a) Einschließen von Hydroxypropylmethylcellulose in der Sonnenschutzzusammensetzung, vorausgesetzt, dass die Hydroxypropylmethylcellulose einen $DS_{Methoxyl}$ von weniger als 1,7 aufweist; oder
b) Auswählen einer Hydroxypropylmethylcellulose mit einem $DS_{Methoxyl}$ von weniger als 1,7 und Inkorporieren der Hydroxypropylmethylcellulose in der Sonnenschutzzusammensetzung.

**Revendications**

**1.** Une composition d'écran solaire, comprenant :

des particules d'écran solaire en oxyde de métal inorganiques sélectionnées parmi de l'oxyde de zinc transparent dans une quantité allant de 0,1 % à 50 % en poids de la composition, du dioxyde de titane transparent dans une quantité allant de 0,1 % à 50 % en poids de la composition, ou des mélanges de ceux-ci ; et
de l'hydroxypropyl méthylcellulose, à condition que l'hydroxypropyl méthylcellulose ait un $DS_{méthoxyl}$ inférieur à 1,7,
dans laquelle l'hydroxypropyl méthylcellulose est présente dans une quantité allant de 0,05 % à 15 % en poids de la composition ;
dans laquelle le terme « transparent » est utilisé ici pour signifier que les particules sont transparentes à l'application.

**2.** La composition d'écran solaire de la revendication 1, dans laquelle les particules d'écran solaire en oxyde de zinc transparentes sont présentes dans une quantité allant de 1 % à 25 %.

**3.** La composition d'écran solaire de la revendication 1, dans laquelle les particules d'écran solaire en dioxyde de titane transparentes sont présentes dans une quantité allant de 1 % à 25 %.

**4.** La composition d'écran solaire de la revendication 1, dans laquelle l'hydroxypropyl méthylcellulose est présente dans une quantité allant de 0,1 % à 5 %.

**5.** La composition d'écran solaire de la revendication 1, dans laquelle l'hydroxypropyl méthylcellulose a un $DS_{méthoxyl}$ inférieur à 1,6, de préférence inférieur à 1,5, de préférence inférieur à 1,4.

**6.** La composition d'écran solaire de la revendication 1, dans laquelle l'hydroxypropyl méthylcellulose a une viscosité à 2 % dans l'eau allant de 1 cps à 100 000 cps, de préférence 15 cps.

**7.** La composition d'écran solaire de la revendication 1, comprenant en outre un humectant.

**8.** La composition d'écran solaire de la revendication 1, comprenant en outre un tensioactif.

**9.** La composition d'écran solaire de la revendication 1, comprenant en outre un émollient.

**10.** La composition d'écran solaire de la revendication 1, comprenant en outre un conservateur.

**11.** La composition d'écran solaire de la revendication 1, comprenant en outre au moins un actif antisolaire organique.

**12.** Un procédé pour renforcer le FPS d'une composition d'écran solaire comprenant des particules d'écran solaire en oxyde de métal inorganiques sélectionnées parmi de l'oxyde de zinc transparent, du dioxyde de titane transparent, ou des mélanges de ceux-ci, comprenant le fait :

    a) d'inclure de l'hydroxypropyl méthylcellulose dans la composition d'écran solaire, à condition que l'hydroxypropyl méthylcellulose ait un $DS_{méthoxyl}$ inférieur à 1,7 ; ou
    b) de sélectionner une hydroxypropyl méthylcellulose avec un $DS_{méthoxyl}$ inférieur à 1,7, et d'incorporer l'hydroxypropyl méthylcellulose dans la composition d'écran solaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61107507 A
- US 5223250 A
- US 5372805 A
- US 5573753 A
- US 5587148 A
- US 5876688 A
- US 5733895 A
- US 7390355 B